Europäisches Patentamt

European Patent Office

Office européen des brevets

(11) Veröffentlichungsnummer: **0 002 501**
**B1**

(12) **EUROPÄISCHE PATENTSCHRIFT**

(45) Veröffentlichungstag der Patentschrift:
**12.08.81**

(21) Anmeldenummer: **78101595.3**

(22) Anmeldetag: **07.12.78**

(51) Int. Cl.³: **C 07 C 21/06,** C 07 C 19/045,
C 07 C 17/34, C 07 C 17/38

(54) Verfahren zur Rückführung von nichtumgesetztem 1.2-Dichloräthan aus der 1.2-Dichloräthanspaltung.

(30) Priorität: **09.12.77 DE 2754891**

(43) Veröffentlichungstag der Anmeldung:
**27.06.79 Patentblatt 79/13**

(45) Bekanntmachung des Hinweises auf die Patenterteilung:
**12.08.81 Patentblatt 81/32**

(84) Benannte Vertragsstaaten:
**BE FR GB IT NL SE**

(56) Entgegenhaltungen:
**DE-A-2 307 376**
**DE-B-2 416 786**
**FR-A-2 028 791**
**FR-A-2 038 347**
**FR-A-2 080 645**
**US-A-4 060 460**

(73) Patentinhaber: **WACKER-CHEMIE GMBH,
Prinzregentenstrasse 22, D-8000 München 22 (DE)**

(72) Erfinder: **Schmidhammer, Ludwig, Dr., Pappelweg 5,
D-8261 Haiming/Marktl (DE)**
Erfinder: **Frey, Hellmuth, Baaderstrasse 2a,
D-8263 Burghausen (DE)**

(74) Vertreter: **Kraus, Walter, Dr. et al, Patentanwälte Dres.
Kraus & Weisert Irmgardstrasse 15,
D-8000 München 71 (DE)**

Verfahren zur Rückführung von nichtumgesetztem
1.2-Dichloräthan aus der 1.2-Dichloräthanspaltung

Vinylchlorid wird in großtechnischem Maßstab durch Pyrolyse von 1.2-Dichloräthan hergestellt. Je höher die Pyrolysetemperatur und damit der Umsatz eingestellt wird, desto mehr unerwünschte Nebenprodukte entstehen bei diesem Spaltprozeß. Das während der Spaltung nicht umgesetzte 1.2-Dichloräthan enthält nach Abtrennung von Chlorwasserstoff und Vinylchlorid bei gleichzeitiger Ausschleusung von in Spurenmengen vorhandenen niedrigsiedenden Nebenprodukten wie 1.3-Butadien, Acetylen, Monovinylacetylen und Methylchlorid, noch 1.1-Dichloräthylen, 1.2-Dichloräthylen, Chloroform, Tetrachlorkohlenstoff, Trichloräthylen, Benzol und vor allem in größeren Mengen 2-Chlorbutadien-1.3 und etwas 1-Chlorbutadien-1.3. Bei der Rückführung des nichtumgesetzten 1.2-Dichloräthans hat es sich als unerläßlich erwiesen, diese Nebenprodukte zu entfernen, um ein rasches Zuwachsen des Spaltofens mit Ruß und Koks zu vermeiden.

Eine Entfernung der genannten Nebenprodukte durch Destillation führt jedoch zur Anreicherung und nachfolgender Polymerisation des Chloroprens in der Destillationskolonne und damit verbundenen Betriebsstörungen.

Aus der französischen Patentschrift 2 080 645 ist bekannt, dem 1.2-Dichloräthan enthaltenden Spaltgas vor der Abtrennung des Vinylchlorids und des Chlorwasserstoffes geringe Mengen Chlor zuzusetzen. Das flüssige Reaktionsgemisch muß dabei nach dem Chlorzusatz eine gewisse Zeit in einem Verweilbehälter verbleiben, bevor es der weiteren Aufarbeitung zugeführt werden kann. Abgesehen von einer nicht vollständigen Beseitigung der oben geschilderten Problematik ist es erforderlich, zur Vermeidung von Korrosion besondere Schutzmaßnahmen zu ergreifen oder Apparaturen aus sehr edlen bzw. sehr teuren Werkstoffen einzusetzen.

Bei einer Chlorzugabe von 0,01 bis 1 Gew.-% bezogen auf das ursprünglich eingesetzte Dichloräthan, gelöst in 1.2-Dichloräthan gemäß der DE-AS 2 307 376 vor der Abtrennung des Vinylchlorids ist es nachteilig, daß ein Teil des zugesetzten Chlors mit dem Vinylchlorid zu wertlosem 1.1.2-Trichloräthan abreagiert, bzw. nicht umgesetztes 1.2-Dichloräthan unter den angegebenen Reaktionsbedingungen teilweise substituiert wird. Eine quantitative Chlorierung des Chloroprens kann nicht beobachtet werden.

Nach der DE-AS 2 416 786 wird bei der Aufarbeitung der Reaktionsprodukte einer thermischen 1.2-Dichloräthanspaltung nach Abtrennung des Chlorwasserstoffs und Vinylchlorids der Kolonnenteil, in dem die hochsiedenden Produkte abgetrennt werden sollen, ohne Anwesenheit eines Katalysators mit Chlor begast. Durch eine damit bedingte Verminderung des Chloroprens kann die Laufzeit des Spaltofens wie auch der Kolonne gesteigert werden, eine quantitative Chloroprenausschleusung, die für eine Spaltofenlaufzeitoptimierung unerläßlich ist, kann jedoch nicht erreicht werden. Die gleichzeitige Anwesenheit von Chlor im Destillationssumpf bei Temperaturen von etwa 100 bis 110°C führt zu merklicher Chlorsubstitution des 1.2-Dichloräthans zu hochsiedendem 1.1.2-Trichloräthan und damit einhergehender Unwirtschaftlichkeit des Verfahrens.

In der FR-PS 2 038 347 wird ein Verfahren für die Chlorierung von Chloropren in nichtumgewandeltes 1.2-Dichloräthan beschrieben. In dieser Patentschrift wird angegeben, daß man dabei einen Katalysator für die Chlorierung von Äthylen verwenden kann. Der einzige dort beschriebene Katalysator ist Eisen(III)-chlorid. Andere Katalysatoren werden in dieser Patentschrift nicht erwähnt.

Die FR-PS 2 028 791 betrifft ein Verfahren zur Chlorierung von Olefinen mit 2 bis 4 Kohlenstoffatomen einschließlich Äthylen und Butadien. Als Katalysatoren für dieses bekannte Verfahren werden Kresolverbindungen erwähnt. Hinsichtlich der Chlorierung von Butadien finden sich keine Hinweise. In Beispiel 5 wird nur angegeben, daß als Reaktionsprodukte hauptsächlich gleiche Mengen von 1.4-Dichlorbuten-2 und 1.2-Dichlorbuten-3 entstehen. Die Verwendung von Kresolderivaten als Katalysatoren bei einem Verfahren zur Entfernung der störenden Nebenprodukte, insbesondere von 1.3-Chlorbutadien-1.3 und 2-Chlorbutadien-1.3, aus dem bei der Pyrrolyse von 1.2-Dichloräthan nach der Abtrennung von Chlorwasserstoff und Vinylchlorid verbleibenden Reaktionsstrom hat nicht nahegelegen, da in der Literatur unzählige Katalysatoren beschrieben werden, denen katalytische Wirksamkeit bei der Chlorierung von Äthylen zugeschrieben wird. Es war überraschend und war nicht naheliegend, daß man bei der Verwendung bestimmter hydroxylgruppenhaltiger Aromaten, die sich von Kresol ableiten, Chloropren quantitativ chlorieren kann und gleichzeitig einen praktisch chlorfreien Reaktionsstrom erhält.

Aufgabe der Erfindung war es somit, ein Verfahren zur Behandlung vor der Rückführung von nichtumgesetztem 1.2-Dichloräthan aus der 1.2-Dichloräthanspaltung zu finden, bei dem störende Nebenprodukte, insbesondere 1-Chlorbutadien-1.3 und 2-Chlorbutadien-1.3 in einfacher und gezielter Weise unter Vermeidung der Nachteile der bisher bekannten Verfahren nahezu quantitativ zu entfernen und somit optimale Laufzeiten für den Pyrolysereaktor und die Aufarbeitungskolonnen zu erreichen.

Gegenstand der Erfindung ist ein Verfahren zur Behandlung vor der Rückführung von nichtumgesetztem 1.2-Dichloräthan aus der 1.2-Dichloräthanspaltung, wobei Chlorwasserstoff und Vinylchlorid entfernt wurden, dadurch gekennzeichnet, daß der verbleibende Rest vor der Rückführung mit hydroxylgruppenhaltigen Aromaten der Art o-, m-Kresol bzw. deren Mono- oder Dichlorderivaten in Mengen von 0,0001 bis 0,01 Gew.-% bezogen auf nichtumgesetztes 1.2-Dichloräthan einzeln oder im Gemisch versetzt einem Chlorierungs- und nachfolgendem Dechlorierungsschritt jeweils bei Temperaturen zwischen 0 und 80° C, vorzugsweise 20 bis 30° C, bei Drücken zwischen 0,5 und 6 bar, unterzogen wird, wobei zur Dechlorierung dem höchstzulässigen Chlorüberschuß von 800 ppm nach dem Chlorierungsschritt äquivalente Mengen Äthylen zugesetzt werden und anschließend das 1.2-Dichloräthan abdestilliert wird.

Eine bevorzugte Ausführungsform des Verfahrens besteht darin, daß man dem Chlor bis zu 1 Vol.-% Sauerstoff beimischt.

Das erfindungsgemäße Verfahren hat den Vorteil, daß kein 1.2-Dichloräthan und praktisch kein Äthylen und kein Chlor verlorengeht. Als überraschend ist zu bezeichnen, daß bereits so geringe Mengen der genannten hydroxylgruppenhaltigen Aromaten zur quantitativen Chlorierung der isomeren Chlorbutadiene ausreicht und nach nur kurzer Verweilzeit in der Chlorierungszone das überschüssige Chlor unmittelbar darauf mit Äthylen entfernt werden kann. Ohne Zugabe der hydroxylgruppenhaltigen Aromaten der genannten Art hingegen setzt sich selbst bei Anwesenheit von Spuren Eisenchlorid als bekanntem Chlorübertragungskatalysator äquimolares Äthylen in der vorgegebenen Verdünnung mit gelöstem freiem Chlor nur zu etwa 40% um, während ein Großteil des Chlors 1.2-Dichloräthan zu 1.1.2-Trichloräthan substituiert.

Nach der Pyrolysereaktion wird das nichtumgesetzte 1.2-Dichloräthan zusammen mit den Verunreinigungen aus dem Sammelgefäß abgeleitet und vermittels geeigneter Gegenstromwärmetauscher auf die beanspruchte Reaktionstemperatur von 0 bis 80° C, vorzugsweise 20 bis 30° C und Drücke zwischen 0,5 und 6 bar gebracht. Die Zugabe der hydroxylgruppenhaltigen Aromaten der Art o-, m-Kresol bzw. deren Mono- oder Dichlorderivate in Mengen von 0,0001 bis 0,1 Gew.-% bezogen auf nichtumgesetztes 1.2-Dichloräthan einzeln oder im Gemisch erfolgt vorteilhaft mittels einer geeigneten Dosierpumpe aus einem Vorratsbehälter, in dem entsprechende Mengen dieser hydroxylgruppenhaltigen Aromaten in 1.2-Dichloräthan gelöst vorliegen. Die Zugabe kann auch vor der bereits erwähnten Konditionierung des nichtumgesetzten 1.2-Dichloräthans zugesetzt werden.

Die nachfolgende selektive Chlorierung der im nichtumgesetzten 1.2-Dichloräthan vorhandenen isomeren Chlorbutadiene kann in einem leeren oder mit Füllkörpern gepackten Reaktionsrohr durchgeführt werden, der Dechlorierungsreaktor ist vorteilhafterweise ein mit Füllkörpern gepacktes Strömungsrohr, um das Äthylen besser zu verteilen. Als Chlorquelle eignet sich verdampftes Flüssigchlor oder Elektrolysechlor dem ggf. so viel Sauerstoff zugemischt ist, daß ein maximaler Sauerstoffgehalt von 1 Vol.-% resultiert. Höhere Sauerstoffgehalte beinhalten die Gefahr, daß sich in nachgeschalteten Pufferbehälter explosive Gasgemische bilden können. Bei der erfindungsgemäßen Verfahrensweise reicht es aus, Reaktionsrohre aus herkömmlichem Kohlenstoffstahl zu verwenden. Teure Spezialstähle sind für diesen Verfahrensschritt nicht notwendig. Die Verweilzeit im Reaktionsrohr soll für die Chlorierungsreaktion zwischen 20 und 50 Sekunden, für die Dechlorierungsreaktion 10 bis 40 Sekunden betragen. Spezielle Durchmesser- zu Längeverhältnisse der Reaktionsrohre sind nicht ausschlaggebend, jedoch ist es von Vorteil, das Durchmesser- zu Längeverhältnis so auszuwählen, daß neben der geforderten Verweilzeit auch eine Durchmischung des Reaktionsgutes im Strömungsrohr stattfindet. Die in der Dechlorierungsreaktion anwesenden Raschigringe bestehen aus Eisen.

Die erfindungsgemäß zugegebenen OHgruppenhaltigen Aromaten werden teilweise chloriert und zusammen mit den übrigen Hochsiedern im Sumpf der Schwersiederkolonne ausgeschleust. Damit ist eine Belastung des Dichloräthankreislaufes bei der Rückführung zur thermischen Spaltungsreaktion nicht gegeben.

Die Zugabemenge Chlor in der Chlorierungszone zur Chlorierung des 1-Chlorbutadien-1.3 bzw. 2-Chlorbutadien-1.3 wird so bemessen, daß nach der Chlorierung ein maximaler Chlorüberschuß von 800 ppm resultiert. Als Erfahrungswerte haben sich ergeben, daß pro Mol im nichtumgesetzten 1.2-Dichloräthan enthaltenen 2-Chlorbutadien-1.3 1,2 bis 1,7 Mol Chlor und pro Mol im nichtumgesetzten 1.2-Dichloräthan enthaltenen 1-Chlorbutadien-1.3 2,1 bis 2,3 Mol Chlor eingesetzt werden müssen. Die im nichtumgesetzten 1.2-Dichloräthan äußerst störend wirkenden Nebenprodukte 1-Chlorbutadien-1.3 und 2-Chlorbutadien-1.3 werden quantitativ zu unschädlichen Hochsiedern aufchloriert, ohne daß eine merkliche Substitution von 1.2-Dichloräthan zu 1.1.2-Trichloräthan einsetzt und ohne daß Äthylchlorid, durch Reaktion zwischen Äthylen und Spuren Chlorwasserstoff in Anwesenheit von Spuren von Eisensalzen, wie sie sich auf der Stahlapparatur ausbilden könnten, gebildet werden.

Beispiel 1

30 t/h nichtumgesetztes 1.2-Dichloräthan mit einem Gehalt von 6 Gew.ppm 1.1.2-Trichloräthan, 10 Gew.ppm Äthylchlorid, 1500 Gew. ppm 2-Chlorbutadien-1.3 und 150 Gew. ppm 1-Chlorbutadien-1.3, im Sumpf der Vinylchlorid-Gewinnungskolonne mit einem Druck von 5 bar und einer Temperatur von 152° C anfallend, werden unter kontinuierlicher Zudosierung von 10 Gew. ppm o-Kresol, gelöst in 1.2-Dichloräthan, mittels einer Kühlvorrichtung auf 30° C abgekühlt und nachfolgend mit 0,843 kmol/h verdampftem Flüssigchlor in einem leeren Reaktionsrohr (Durchmesser: 250 mm, Länge: 5000 mm) begast, wodurch am Austritt der Chlorierungszone im nichtumgesetzten 1.2-Dichloräthan die isomeren Chlorbutadiene auf einen Wert kleiner 1 Gew. ppm entfernt werden, während die 1.1.2-Trichloräthan-Konzentration bei einem Überschuß von 460 Gew. ppm freiem Chlor auf nur 176 Gew. ppm ansteigt. Unmittelbar nach Verlassen der Chlorierungszone gelangt das nichtumgesetzte 1.2-Dichloräthan in ein mit Fe-Raschigringen gefülltes Strömungsrohr (Durchmesser 220 mm, Länge 3800 mm), in dem es mit 0,1944 kmol/h Äthylen begast wird, wobei das vorhandene freie Chlor nahezu quantitativ zu 1.2-Dichloräthan umgesetzt wird, ohne daß der Äthylchloridspiegel im nichtumgesetzten 1.2-Dichloräthan ansteigt. Das die Dechlorierung verlassende nichtumgesetzte 1.2-Dichloräthan enthält nur noch 2 Gew. ppm freies Chlor entsprechend einem Äthylenumsatz von 99,6%.

### Beispiel 2
### (Vergleichsbeispiel)

Analog Beispiel 1 werden 30 t/h nichtumgesetztes 1.2-Dichloräthan mit denselben Ausgangsverunreinigungen in Abwesenheit von o-Kresol nacheinander mit 1,247 kmol/h verdampftem Flüssigchlor und 0,1944 kmol/h Äthylen unter denselben Reaktionsbedingungen behandelt, obwohl sich dadurch in der Chlorierungszone ein Überschuß von 460 Gew. ppm freiem Chlor einstellt, enthält das nichtumgesetzte 1.2-Dichloräthan nach der Chlorierungszone noch 20 Gew. ppm 2-Chlorbutadien neben 2000 Gew. ppm 1.1.2-Trichloräthan, das durch Chlorsubstitution von 1.2-Dichloräthan entstanden ist, während der Dechlorierungszone der 1.1.2-Trichloräthanspiegel gar noch auf 2140 Gew. ppm ansteigt und das die Dechlorierungszone verlassende nichtumgesetzte 1.2-Dichloräthan außer 30 Gew. ppm Äthylchlorid, entstanden durch Reaktion zwischen Äthylen und Chlorwasserstoff, noch 200 Gew. ppm freies Chlor neben etwa 110 Gew. ppm Äthylen enthält (entsprechend einem Äthylenumsatz von 40%, welches im drucklosen Pufferbehälter zum größten Teil ausgast und somit verloren ist). Das noch vorhandene freie Chlor baut sich in einer Zeitreaktion durch Substitution mit 1.2-Dichloräthan bzw. durch Chlorierung des restlichen 2-Chlorbutadien-1.3 noch teilweise bis auf etwa 100 Gew. ppm ab, wodurch der 1.1.2-Trichloräthanspiegel im nichtumgesetzten 1.2-Dichloräthan im Pufferbehälter auf ca. 2300 Gew. ppm ansteigt. Darüber hinaus entstehen durch die Anwesenheit von freiem Chlor und größeren Mengen Chlorwasserstoff, der bei der substituierenden Chlorierung von 1.2-Dichloräthan anfällt, schwerwiegende Umwelt- und Korrosionsprobleme in den Abgasen dieses Pufferbehälters, die eine Wäsche der Tankabgase erforderlich machen.

### Beispiel 3

20 t/h nichtumgesetztes 1.2-Dichloräthan aus einer thermischen Spaltung von 1.2-Dichloräthan in Vinylchlorid und Chlorwasserstoff mit einem Gehalt von 5 Gew. ppm 1.1.2-Trichloräthan, 7 Gew. ppm Äthylchlorid, 1000 Gew. ppm 2-Chlorbutadien-1.3 und 120 Gew. ppm 1-Chlorbutadien-1.3 werden analog Beispiel 1 kontinuierlich mit 15 Gew. ppm m-Kresol versetzt und auf 32°C abgekühlt. Anschließend begast man das nichtumgesetzte 1.2-Dichloräthan in demselben leeren Reaktionsrohr wie in Beispiel 1 beschrieben mit 0,404 kmol/h Elektrolysechlor, welches ca. 0,6 Vol.-% Sauerstoff enthält, wodurch am Austritt der Chlorierungszone im nichtumgesetzten 1.2-Dichloräthan die isomeren Chlorbutadiene auf einen Wert kleiner 1 ppm entfernt werden, während die 1.1.2-Trichloräthankonzentration bei einem Überschuß von 350 Gew. ppm freiem Chlor auf nur 165 Gew. ppm ansteigt. Unmittelbar nach Verlassen der Chlorierungszone wird das nichtumgesetzte 1.2-Dichloräthan in demselben Strömungsrohr, wie im Beispiel 1 beschrieben, mit 0,099 kmol/h Äthylen begast, wobei das vorhandene freie Chlor nahezu quantitativ zu 1.2-Dichloräthan umgesetzt wird, ohne daß der ursprüngliche Äthylchlorid-Spiegel ansteigt bzw. ohne eine weitere Vermehrung von Substitutionsprodukten des 1.2-Dichloräthans. Das die Dechlorierungszone verlassende nichtumgesetzte 1.2-Dichloräthan enthält nur noch 1 Gew. ppm freies Chlor entsprechend einem Äthylenumsatz von 99,99%.

### Beispiel 4
### (Vergleichsbeispiel)

Analog Beispiel 3, aber ohne Zudosierung von m-Kresol, werden 20 t/h nichtumgesetztes 1.2-Dichloräthan mit denselben Verunreinigungen nacheinander mit 0,565 kmol/h Elektrolysechlor und 0,384 kmol/h Äthylen begast. Das nichtumgesetzte 1.2-Dichloräthan enthält nach der Chlorierungszone neben 550 Gew. ppm freiem Chlor Gew. ppm 1.1.2-Trichloräthan und 40 Gew ppm 2-Chlorbutadien-1.3, welches in der nachfolgenden Dechlorierungszone nicht mehr entfernt werden kann, da durch entsprechend vermehrte Zugabe von Äthylen über die Stöchiometrie hinaus das freie Chlor wegreagiert worden ist. Im Ablauf der Dechlorierungszone findet man neben 5 Gew. ppm freiem Chlor 25 Gew. ppm Äthylchlorid, 960 Gew. ppm 1.1.2-Trichloräthan und 0,238 kmol/h Äthylen entsprechend einem Umsatz von 37,6%, welches im drucklosen Pufferbehälter für nichtumgesetztes 1.2-Dichloräthan zum größten Teil ausgast, also verlorengeht. Um das Chlorpren quantitativ

entfernen zu können, muß die Einleitung des Äthylens hinter der Dechlorierungszone erfolgen, um für die Reaktion zwischen Chlor und 2-Chlorbutadien-1.3 mehr Verweilzeit zu schaffen.

### Beispiel 5

Analog Beispiel 1 werden 30 t/h nichtumgesetztes 1.2-Dichloräthan gleicher Zusammensetzung in Gegenwart von 5 Gew. ppm o-Kresol und 5 Gew. ppm m-Kresol bei 20°C und einem Druck von 3 bar mit 0,927 kmol/h verdampftem Flüssigchlor und 0,302 kmol/h Äthylen nacheinander in Berührung gebracht. Das die Dechlorierungszone verlassende nichtumgesetzte 1.2-Dichloräthan enthält folgende Verbindungen:

| | | | |
|---|---|---|---|
| 10 | Gew. | ppm | Äthylchlorid |
| <1 | Gew. | ppm | 2-Chlorbutadien-1.3 |
| <1 | Gew. | ppm | 1-Chlorbutadien-1.3 |
| 74 | Gew. | ppm | 1.1.2-Trichloräthan |
| 1 | Gew. | ppm | freies Chlor |
| | kein | | Äthylen |

### Patentansprüche

1. Verfahren zur Rückführung von nichtumgesetztem 1,2-Dichloräthan aus der 1,2-Dichloräthanspaltung, wobei Chlorwasserstoff und Vinylchlorid entfernt wurden, dadurch gekennzeichnet, daß der nach der Abtrennung von Chlorwasserstoff und Vinylchlorid verbleibende Rest vor der Rückführung mit o-, m- Kresol bzw. deren Mono- oder Dichlorderivaten als hydroxylgruppenhaltigen Aromaten in Mengen von 0,0001 bis 0,01 Gew.-%, bezogen auf nichtumgesetztes 1,2-Dichloräthan, einzeln oder im Gemisch versetzt, einem Chlorierungs- und nachfolgenden Dechlorierungsschritt jeweils bei Temperaturen zwischen 0 und 80°C, vorzugsweise 20 bis 30°C, bei Drücken zwischen 0,5 und 6 bar, unterzogen wird, wobei zur Dechlorierung dem höchstzulässigen Chlorüberschuß von 800 ppm nach dem Chlorierungsschritt äquivalente Mengen Äthylen zugesetzt werden und anschließend das 1,2-Dichloräthan abdestilliert wird.

2. Verfahren nach Anspruch 1, dadurch gekennzeichnet, daß dem Chlor bis zu 1 Vol.-% Sauerstoff beigemischt wird.

### Claims

1. A process for recycling unreacted 1,2-dichloroethane from the splitting of 1,2-dichloroethane, hydrogen chloride and vinyl chloride being removed, characterised in that, before recycling, the residue left after the removal of hydrogen chloride and vinyl chloride is subjected to a chlorination and subsequent dechlorination step with o-, m-cresol or their mono- or di-chloro derivatives as aromatic compounds containing hydroxyl groups in quantities of from 0.0001 to 0.01% by weight, based on unreacted 1,2-dichloroethane, either individually or in admixture at temperatures in the range from 0 to 80°C and preferably at temperatures in the range from 20 to 30°C and under pressures of from 0.5 to 6 bar, quantities of ethylene equivalent to the maximum permitted excess of chlorine of 800 ppm after the chlorination step being added for dechlorination, after which the 1,2-dichloroethane is distilled off.

2. A process as claimed in Claim 1, characterised in that up to 1% by volume of oxygen is added to the chlorine.

### Revendications

1°- Procédé pour le recyclage de 1,2-dichloroéthane n'ayant pas réagi, provenant de la dissociation du 1,2-dichloréthane, tandis que l'acide chlorhydrique et le chlorure de vinyle sont éliminés, lequel procédé est caractérisé en ce que l'on ajoute au reste qui subsiste après la sèparation de l'acide chlorhydrique et du chlorure de vinyle et avant le recyclage, de l'o-drésol, du m-crésol ou leurs désol ou leurs dérivés monoou dichlorés, seuls ou en mélanges, en tant qu'hydrocarbures aromatiques contenant des groupes hydroxyles, à raison de 0,0001 à 0,01% en poids rapporté au 1,2-dichloréthane n'ayant pas réagi, en ce que l'on soumet le mélange à une étape de chloration suivie d'une étape de déchloration, chacune à des températures comprises entre 0 et 80°C, et de préférence entre 20 et 30°C, sous des pressions comprises entre 0,5 et 6 bars, tandis que des quantités équivalentes d'éthylène sont ajoutées, pour réaliser la déchloration, lesquelles quantités sont équivalentes à l'excès de chlore maximum admissible de 800 ppm, après l'étape de chloration, après quoi l'on élimine le 1,2-dichloréthane par distillation.

2°- Procédé selon la Revendication 1, caractérisé en ce qu'on mélange jusqu'à 1% en volume d'oxygène au chlore.